# FASCICULE DE BREVET EUROPEEN

(11) **EP 0 991 663 B1**
(45) Date de publication et mention de la délivrance du brevet: **26.09.2007**
(21) Numéro de dépôt: 98940318.3
(22) Date de dépôt: 22.07.1998
(51) Int. Cl.: C07K 14/35, C12N 15/12, C12N 15/11, C12N 1/36, C12N 1/21, A61K 48/00, G01N 33/53

(54) **SOUCHES DE MYCOBACTERIUM DONT LE GENE ERP EST MODIFIE ET COMPOSITION VACCINALE LA CONTENANT**
MYCOBACTERIUM STÄMME MIT EINEM MODIFIZIERTEN ERP GEN UND ENTHALTENDE IMPFSTOFFZUSAMMENSETZUNG
MYCOBACTERIUM STRAIN WITH MODIFIED ERP GENE AND VACCINE COMPOSITION CONTAINING SAME

(30) Priorité: 22.07.1997 FR 9709303
(43) Date de publication de la demande: 12.04.2000
(73) Titulaire: INSTITUT PASTEUR, 75015 Paris (FR)
(72) Inventeur: GICQUEL, Brigitte, F-75014 Paris (FR); BERTHET, François-Xavier, B-1180 Uccle (BE)
(74) Mandataire: Warcoin, Jacques
(86) Numéro de dépôt international: PCT/FR1998/001627
(87) Numéro de publication internationale: WO 1999/005168

(56) Documents cités:
- WO-A-96/07745
- WO-A-96/23885
- V. PELICIC ET AL.,: "Positive selection of allelic exchange mutants in Mycobacterium bovis BCG" FEMS MICROBIOLOGY LETTERS, vol. 144, 1996, pages 161-166, XP002054672 AMSTERDAM, NL cité dans la demande
- F-X. BERTHET ET AL.,: "Characterization of the Mycobacterium tuberculosis erp gene encoding a potential cell surface protein with repetitive structures" MICROBIOLOGY, vol. 141, no. 9, 1995, pages 2123-2130, XP002061531 READING, GB cité dans la demande
- E.M. LIM ET AL.,: "Identification of Mycobacterium tuberculosis DNA sequences encoding exported proteins, using phoA gene fusions" JOURNAL OF BACTERIOLOGY, vol. 177, no. 1, 1995, pages 59-65, XP000560419
- V. PELICIC ET AL.,: "Efficient allelic exchange and transposon mutagenesis in Mycobacterium tuberculosis" PROCEEDINGS OF THE NATIONAL ACADEMY OF SCIENCES, vol. 94, 1 septembre 1997, pages 10955-10960, XP002054882 WASHINGTON, DC, US

## Description

L'invention concerne une souche de *Mycobacterium* dont le gène *erp* est modifié et la composition vaccinale la contenant.

La tuberculose est une maladie infectieuse causée dans la plupart des cas par l'inhalation de bactéries appartenant au complexe d'espèce *Mycobacterium tuberculosis (M. africanum, M. bovis, M. tuberculosis*)*.* Avec huit millions de nouveaux cas humains annuels causant trois millions de décès au niveau mondial, la tuberculose demeure un problème majeur de santé publique (Sudre et al., 1992). La découverte d'antibiotiques efficaces (Streptomycine, Isoniazide, Rifampicine,...) semblait permettre l'éradication de cette maladie. Cependant, on estime qu'actuellement seulement 50 % des malades sont dépistés et reçoivent un traitement. Ce traitement est souvent inapproprié ou mal suivi et conduit à l'apparition d'un nombre croissant de souches résistantes aux antibiotiques et même polychimiorésistantes (Dooley et al., 1992). Dans ce contexte, la mise au point d'une prophylaxie vaccinale apparaît comme une solution privilégiée pour le contrôle et l'éradication de la tuberculose.

Le fait d'utiliser une bactérie pathogène atténuée en tant que composant d'un vaccin a été largement décrit et mis en oeuvre dans l'art antérieur. Les méthodes pour obtenir de telles bactéries atténuées comprennent la sélection au hasard de mutants induits chimiquement ou par irradiation, ou la production de bactéries recombinantes d'origine pathogène dans lesquelles un gène impliqué dans une voie métabolique a été inactivé par génie génétique.

Straley et al. (1984) ont étudié la survivance de mutants avirulents de *Yersinia pestis* qui sont défectueux dans une ou plusieurs voies métaboliques.

Noriega et al. (1994) ont fabriqué par génie génétique une souche de *Shigella* orale destinée à être utilisée en tant que prototype vaccinal par l'introduction de délétions dans un gène (*aroA*) codant pour une protéine impliquée dans une voie métobolique d'un acide aminé arômatique et ils ont démontré que les souches de *Shigella* résultantcs recombinantes défectives étaient capables d'induire des anticorps protecteurs contre le pathogène de type sauvage.

Un travail important a également été réalisé utilisant *Salmonella* comme modèle. Voir par exemple les rapports de Hoiseth et al. (1981), Levine et al. (1987), Oyston et al (1995) et Curtiss (1990).

Cependant, des travaux similaires n'ont pas encore été réalisés pour *Mycobacterium tuberculosis,* l'agent étiologique de la tuberculose (TB), qui infecte un tiers de la population mondiale et tue trois millions de personnes par an. La tuberculose est la plus importante cause de mortalité dans le monde occasionnée par un ensemble d'organismes infectieux (Bloom et Murray, 1992) regroupés sous l'appellation «complexe *M. tuberculosis*»*.* Selon le WHO, plus de personnes sont décédées de la tuberculose en 1995 qu'au cours d'une quelconque année antérieure. Il a été estimé que jusqu'à un demi milliard de personnes souffriront de tuberculose dans les 50 prochaines années. Cependant, en dépit de son importance, les déterminants génétiques de la virulence et de persistance de *M. tuberculosis* restent faiblement caractérisés.

En effet, la virulence des mycobactéries pathogènes est associée à leur aptitude à croître et persister au niveau intracellulaire. Les bactéries du complexe *M. tuberculosis* parasitent les cellules phagocitaires à l'intérieur desquelles elles résident et se multiplient dans un compartiment vacuolaire spécialisé, appelé le phagosome. Les phagosomes contenant des *M*. *tuberculosis* vivants n'acidifient pas et échappent à la fusion avec les lysosomes. Les mécanismes par lesquels les *M. tuberculosis* rendent leur phagosome plus hospitalier restent inconnus et les gènes mycobactériens affectant leur croissance et leur multiplication intracellulaire sont activement recherchés.

L'extrême difficulté à créer des mutants définis de *M. tuberculosis,* soit par échange allélique ou par mutagénèse par transposon, a empêché l'identification de ces facteurs de virulence selon les postulats de Koch (Falkow, 1988 ; Jacobs, 1992). Des stratégies génétiques alternatives ont plutôt été utilisées, incluant la complémentation d'une bactérie non pathogène (Arruda et al., 1993) et des mutants avirulents spontannés avec des bibliothèques d'ADN chromosomiques de *M. tuberculosis* (Pascopella et al. 1994) et *M*. *bovis* (Collins et al., 1995) virulents. Bien que ces études ait identifié des gènes potentiellement impliqués dans l'entrée à l'intérieur des cellules épithéliales et conférant un avantage de croissance *in vivo,* la grande majorité des gènes mycobactériens impliqués dans la virulence et la survie dans l'organisme hôte restent inconnus. Le développement de systèmes de mutagène efficace est donc la priorité pour la génétique mycobactérienne.

Une méthode pour la création de mutants est la mutagénèse par échange allélique. Récemment, des échanges alléliques ayant lieu avec une faible fréquence ont été mis en évidence chez des bactéries du complexe *M. tuberculosis* utilisant un vecteur à effet suicide (Reyrat et al., 1995 ; Azad et al., 1996) et de nouveaux protocoles permettant une détection plus facile des mutants par échange allélique ont également été développés (Norman et al., 1995 ; Balasubramamian et al., 1996 ; Pelicic et al., *FEMS Microbiol. Lett.* 1996). Cependant, la détection d'événement par échange allélique très rare est empêchée par les faibles efficacités de transformations et la fréquence importante de recombinaisons illégitimes. Ainsi, de nombreux gènes de *Mycobacterium* restent encore réfractaires à l'échange allélique au moyen des technologies disponibles.

Plus particulièrement, les systèmes de mutagénèse par échange allélique requièrent la mise en oeuvre de procédés plus efficaces. Les problèmes rencontrés peuvent être contournés par l'utilisation d'un vecteur replicatif qui est efficacement perdu de manière conditionnelle. La possibilité d'introduire de tels vecteurs permet d'éviter les problèmes résultant des faibles efficacités de transformation. Ainsi, dans des conditions de contre-sélection, les clones qui contiennent encore le vecteur sont éliminés permettant ainsi la détection d'événements génétiques très rares. Un tel système a récemment été développé. Utilisant un vecteur replicatif dans certaines conditions qui est perdu à 39°C chez *M. smegatis,* la première bibliothèque de mutants insertionnels mycobactériens a été construite dans cette souche modèle à croissance rapide (Guilhot et al., 1994). Cependant, les vecteurs thermosensibles utilisés ne sont que faiblement thermosensibles dans des mycobactéries à croissance lente du complexe *M. tuberculosis* et donc ne peuvent pas être utilisés dans ces espèces pour la mutagénèsc par échange allélique (donnée non publiée).

Les inventeurs ont réussi à affecter la virulence et la persistance des souches de *Mycobacterium* dans les cellules hôtes.

Ils ont en effet réalisé une souche de *Mycobacterium* dont un gène a été modifié de façon à en atténuer la virulence.

Par gène modifié, on entend un gène ayant subi une modification abolissant la production de la protéine correspondante ou permettant la production d'une protéine différant d'au moins 20 % en terme d'activité par rapport à la protéine naturelle.

Le BCG (Bacille de Calmette et Guérin), une souche avirulente dérivée de *M. bovis,* est très utilisée de par le monde comme vaccin contre la tuberculose. Cependant si le BCG peut être administré sans problème à des individus ne présentant pas de déficience immunitaire, il n'en est pas de même chez les individus immunodéprimés tels que les personnes infectées par le virus du SIDA, les personnes ayant subi une greffe de moëlle, les personnes atteintes d'un cancer, ou les personnes affectées dans le fonctionnement d'un des composants du système immunitaire.

C'est la raison pour laquelle la présente invention concerne une souche de *Mycobacterium* dont la persistence est limitée

Le gène modifié dans la souche de *Mycobacterium* conforme à l'invention est le gène *erp.* Il peut également s'agir d'un gène possédant une homologie (d'au moins 80 %) par complémentation avec le gène *erp.*

L'analyse de la séquence protéique déduite du gène *erp* montre que celui-ci code pour une protéine dont la masse moléculaire calculée est de 28kDa. La présence d'une séquence signal d'exportation en situation N-terminal ainsi que l'existence d'une région hydrophobe en C-terminal suggèrent que la molécule puisse être ancrée dans la membrane plasmique ou localisée à la surface des bacilles. De plus, la région centrale de la protéine comporte deux régions répétées composées chacune de 6 exemplaires d'un motif P(G/A)LTS positionnés en tandem. Cette organisation rappelle celle trouvée chez de nombreuses protéines de surface, associées au peptidoglycane chez les bactéries à Gram positif et chez *Plasmodium.*

Une méthodologie génétique permettant la sélection et l'identification de fragments d'ADN codant pour des protéines exportées, a été récemment adaptée à *M. tuberculosis* au laboratoire. Ce système repose sur la réalisation de banques de fragments d'ADN de *M. tuberculosis* fusionnés avec le gène de la phosphatase alcaline *(phoA)* de E. *coli,* dépourvu de ses signaux d'expression et d'exportation. La phosphatase alcaline (PhoA) ne possède d'activité enzymatique détectable qu'après exportation au travers de la membrane plasmique. En utilisant ce système, plusieurs fragments d'ADN permettant l'exportation de PhoA chez les mycobactéries ont été sélectionnés en présence de substrat chromogène, et partiellement séquencés. Une des fusions portée par le plasmide recombinant pExp53, présente des similarités de séquence avec un gène de *M. leprae* qui code pour une protéine de 28kDa, potentiellement localisée à la surface du bacille. De plus, cette protéine est un antigène majeur de *M. leprae,* reconnu par les sera des patients lépreux lépromateux (WO 9607745). Nous avons de plus déterminé par des expériences d'hybridation moléculaire que le gène *erp* est unique dans le génome de *M. tuberculosis* et qu'il est aussi présent dans le génome des autres membres de ce complexe d'espèce (M. *africanum, M. bovis, M. bovis* BCG).

Afin de permettre l'étude de ERP et de confirmer sa localisation à la surface, des anticorps spécifiques anti-ERP ont été produits. Pour cela, la protéine ERP en fusion avec la protéine liant le maltose (MalE/MBP) ou en fusion avec un peptide C-terminal contenant 6 résidus histidine a été produite. Cette stratégie a permis d'obtenir en grande quantité les protéines ERP-MalE et ERP(His)6 recombinantes, exprimées dans *Escherichia coli.* La purification de ces molécules a été réalisée en utilisant les techniques de chromatographie d'affinité sur résine d'amylose (système MalE) ou de nickel chélaté (système Histidine). Le poids moléculaire relatif déterminé en électrophorèse PAGE-SDS est de 36K. La différence avec le poids moléculaire théorique peut être attribuée a un retard de migration électrophorétique dû à la forte teneur (15%) en résidus proline. Un protocole d'immunisation de lapins, à l'aide des chimères ERP-MalE et ERP(His)6 purifiées, a permis d'obtenir des sera polyclonaux à haut titre, permettant la détection spécifique de la protéine ERP.

A l'aide des anti-séra obtenus chez le lapin, la localisation de la protéine ERP chez *Mycobacterium tuberculosis* a été précisée. Des observations en microscopie électronique après un immunomarquage à l'or colloïdal ont permis de détecter la présence de la protéine ERP à la surface de bacilles tuberculeux issus de culture *in vitro.* Ainsi, la protéine ERP est susceptible de présenter à la surface des mycobactérics des épitopcs d'autres antigènes et des fins vaccinales ou thérapeutiques. De plus, des expériences similaires ont permis de mettre en évidence la protéine ERP dans des macrophages murins infectés par *M. tuberculosis.*

Afin d'analyser la fonction de la protéine ERP, une souche de BCG dans laquelle le gène *erp* a été modifié par échange allélique a été construite. La survie de cette souche en comparaison avec la souche sauvage a été analysée dans le modèle souris. Il a été mis en évidence que la mutation du gène *erp* affecte sévèrement la persistence de *M*. *bovis* BCG. Cette diminution de la persistance est observée dans tous les organes testés (Rate, foie, Poumons). Outre le rôle du gène dans le processus de survie du BCG, ces observations impliquent que le gène *erp* soit exprimé durant la phase de croissance *in vivo* chez l'hôte.

Plus particulièrement, la modification du gène *erp* est réalisée par mutation, insertion, délétion ou substitution ; la modification d'au moins une paire de base est suffisante.

Selon un mode de réalisation avantageux de la souche conforme à l'invention, le gène *erp* est modifié par insertion d'un nucléotide ou polynucléotide qui peut être un gène de sélection. Ce gène peut notamment coder pour la résistance à un antibiotique tel que la kanamycine, la spectinomycine ou l'hygromycine.

Les souches de *Mycobacterium* préférées sont celles appartenant au genre *Mycobacterium,* préférentiellement au complexe de *Mycobacterium tuberculosis* et plus préférentiellement encore à l'espèce *Mycobacterium tuberculosis* ou à l'espèce *Mycobacterium bovis.*

La présente invention concerne plus particulièrement la souche BCG *erp::Kn* également appelée BCG *erp: :aph* (CNCM No 1- 1896) ou un variant incapable d'exprimer le produit du gène *erp* actif ainsi que la souche *M. tuberculosis* H37Rv *erp : : aph* (CNCM No 1-2048) ou un variant incapable d'exprimer le produit du gène.

L'invention concerne également une souche de *Mycobacterium* dont le gène *erp* est modifié et qui est capable de produire, suite à des événements de recombinaison, des épitopes ou des déterminants antigéniques susceptibles d'immuniser et/ou protéger contre des agents pathogènes tels que des agents infectieux ou des gènes de cancer, ou de produire des molécules conduisant à une modulation du système immunitaire telles que les cytokines, les chémokines, des récepteurs solubles de molécules interagissant avec des agents conduisant à une pathologie ou des inducteurs des réponses immunitaires tels que IL2, IL4, IL10 ou IL12 (chez l'homme ou l'animal).

La présente invention concerne donc également une souche de *Mycobacterium* telle que précédemment décrite capable en outre d'exprimer un polynucléotide codant pour un antigène de mycobacterie d'une autre espèce que celle à laquelle ladite souche appartient. Le polynucléotide en question pouvant être étranger au genre *Mycobacterium.*

L'invention a également pour objcct un polynucléotide purifié comprenant un gène *erp* modifié et un fragment d'au moins 60 nucléotides correspondant à tout ou partie d'un gène codant pour un antigène exporté du genre *Mycobacterium* ou codant pour un antigène étranger au genre *Mycobacterium.*

La modification du gène *erp* peut être obtenue par exemple par addition, insertion ou modification de nucléotides. Dans le cadre de l'invention, la sélection de la souche de *Mycobacterium* dont le gène *erp* est ainsi modifié peut être réalisée par amplification génique et séquençage nucléotidique ou RFLP de la région nucléique mutée dans ladite souche isolée sur gélose selon le protocole de contre-sélection en présence de sucrose (Pelicic et al., 1996) par exemple. Une alternative consiste à effectuer des hybridations dans des conditions de fortes stringcnccs (Berthet et al., 1995) caractérisées par l'utilisation d'une sonde correspondant à tout ou partie du gène *erp* modifié génétiquement mais ayant conservé au moins 20 % de son activité et s'hybridant préférentiellement avec tout ou partie du gène modifié présent dans la souche recherchée.

La modification du gène *erp* peut également être réalisée au moyen d'un vecteur recombinant comprenant le gène *erp* inactivé. Ce vecteur est utilisé pour la transformation d'une souche de *Mycobacterium* et doit permettre un échange allélique avec le gène *erp* sauvage dans le but de le modifier.

Avantageusement, le vecteur conforme à l'invention comprend une origine de replication thermosensible chez les mycobactéries. Il peut comprendre également le gène de contre-sélection *sacB* avec, de façon facultative un gène permettant une sélection positive tel qu'un gène codant pour la résistance à un antibiotique.

La modification du gène *erp* dans le vecteur conforme à l'invention peut être réalisée comme décrit ci-dessus.

Plus particulièrement, ledit vecteur correspond au plasmide recombinant pIPX56 (CNCM No I-1895). En effet, ce plasmide consiste en un vecteur de clonage navette *E. coli* - mycobactéries du type pPR27 (déposé à la CNCM sous le numéro 1-1730) comportant une origine de réplication thermosensible chez les mycobactéries, le gène de contre-sélection *sacB* et conferrant la résistance à la gentamycine. Dans le plasmide pIPX56, une insertion de 5,1 kb d'un fragment PstI a été réalisée au niveau du site unique Pstl de pPR27. Ce fragment de 5,1 kb correspond à un fragment d'ADN de 3,9 kb de *M. tuberculosis* comportant le gène *erp,* au niveau duquel a été inséré une cassette (1,2 kb) conférant la résistance à la kanamycine. Ce plasmide permet donc d'effectuer des expériences d'échange allélique au niveau du locus *erp* chez les mycobactéries.

Dans le cadre de la présente invention, il est également intéressant de pouvoir disposer d'un vecteur dérivé de pIPX56 comprenant le gène *erp* non modifié.

L'invention a donc également pour objet l'utilisation d'un vecteur recombinant tel que décrit ci-dessus pour la préparation d'une souche de *Mycobacterium* conforme à l'invention par échange allélique.

Un autre objet de l'invention est un procédé pour la production d'une souche de *Mycobacterium* telle que ci-dessus décrite comprenant les étapes de :
- transformation avec un vecteur tel que décrit ci-dessus d'une souche de *Mycobacterium* propagée à une température permissive,
- mise en culture des colonies résultant de la transformation sur un milieu supplémenté avec un produit de sélection et du saccharose,
- isolement de la souche recombinante.

Selon un mode de réalisation avantageux du procédé conforme à l'invention, le produit de sélection est un antibiotique tel que la kanamycine, la spectinomycine ou l'hygromycine.

A titre d'exemple, une souche de *Mycobacterium* recombinant est produite conformément à l'invention comme suit :
a) le plasmide pIPX56 est introduit par électroporation dans une souche du complexe *Mycobacterium tuberculosis* propagée à température permissive (32°C). Cette étape permet de disposer d'une population de bactéries dans laquelle chaque individu possède plusieurs copies de la cassette *erp::Kn ;*
b) une colonie résultant de la transformation est cultivée en milieu liquide à 32°C durant 10 jours, puis la culture est ensemencée sur des boîtes contenant de la Kanamycine (50 mg/ml) et du saccharose 2 % (poids/vol.) incubées à température non permissive à 39°C. Cette étape permet d'enrichir en événements de double recombinaison homologue par contre-sélection et élimination des intégrations de vecteurs (simple recombinaison homologue ou recombinaison illégitime).

L'invention concerne également une composition immunogène comprenant une souche de *Mycobacterium* conforme à l'invention ou obtenue par la mise en oeuvre du procédé ci-dessus mentionné.

Elle concerne également une composition vaccinale comprenant une souche de *Mycobacterium* conforme à l'invention ou obtenue par la mise en oeuvre du procédé ci-dessus mentionné, en combinaison avec au moins un excipient pharmaceutiquement compatible.

Cette composition vaccinale est destinée à l'immunisation des humains et des animaux contre une souche pathogène de mycobactéries et comprend une composition immunogène telle que décrite ci-dessus en combinaison avec un excipient pharmaceutiquement compatible (tel qu'un tampon salin), de façon facultative en combinaison avec au moins un adjuvant de l'immunité tel que l'hydroxyde d'aluminium ou un composé appartenant à la famille du peptide muramyl.

Pour obtenir un effet adjuvant pour le vaccin, de nombreuses méthodes prévoient l'utilisation d'agents tels que l'hydroxyde ou le phosphate (alun) d'aluminium, communément utilisés en tant que solution titrant 0,05 à 0,01 % dans un tampon salin de phosphate, mélangés avec des polymères synthétiques de sucre (Carbopol) en que solution à 0,25 %. Un autre composé adjuvant convenable est le DDA (bromure 2 diméthyldioctadécylammonium), ainsi que des substances immuno-modulantes telles que les lymphokines (par exemple gamma-1FN, IL-1, IL-2 et IL-12) ou également des composés inducteurs de gamma-IFN tels que le poly I:C.

La composition vaccinale conforme à la présente invention est de façon avantageuse préparée sous forme injectable, pour une administration orale ou par inhalation, soit en solution liquide soit en suspension ; des formes solides convenables destinées à être mises en solution ou en suspension liquide avant l'injection peuvent également être préparées.

De plus, la composition vaccinale peut contenir des composants mineurs de substance auxiliaire telle que des agents mouillant ou émulsifiant, des agents tamponnant le pH ou des adjuvants qui stimulent l'efficacité des vaccins.

Les compositions vaccinales de l'invention sont administrées d'une façon compatible avec la formulation de dosage et en une quantité thérapeutiquement efficace et immunogène. La quantité à administrer dépend du sujet à traiter y compris par exemple la capacité individuelle de son système immunitaire à induire une réponse immunitaire.

Le dosage du vaccin dépendra de la voie d'administration et variera selon l'âge du patient à vacciner et dans un moindre degré de la taille de ce patient. De façon préférentielle, la composition vaccinale selon la présente invention est administrée par une voie intradermique en une seule fois ou par voie orale ou par aérosol.

Dans certains cas, il sera nécessaire de procéder à de multiples administrations de la composition vaccinale conforme à la présente invention sans toutefois généralement dépasser six administrations, de préférence quatre vaccinations. Les administrations successives se feront normalement avec un intervalle de 2 à 12 semaines, préférentiellement de 3 à 5 semaines. Des rappels périodiques à des intervalles de 1 à 5 ans, préférentiellement 3 ans, sont souhaitables pour maintenir le niveau désiré de l'immunité protectrice.

L'invention concerne également une méthode de diagnostic permettant de discriminer entre des individus d'une part ayant été vaccinés à l'aide d'une souche de *Mycobacterium* ne produisant plus ERP actif et d'autre part ceux ayant subis une infection naturelle ou une vaccination à l'aide d'une souche produisant la protéine ERP naturelle.

En effet, les individus ayant subis une vaccination par une souche de *Mycobacterium* ne produisant plus la protéine ERP naturelle peuvent être distingués par l'absence à partir d'un échantillon biologique, tel que par exemple le sérum, d'anticorps dirigés contre ERP et/ou par l'absence de réactivité T (mesurée par exemple au cours d'un test de prolifération ou d'un test de sécrétion des cytokines ou de test CTL) contre la protéine ERP purifiée. Une alternative consiste également à rechercher une réactivité différentielle à l'aide d'anticorps dirigés contre la partie non modifiée de la protéine ERP naturelle en comparaison avec la partie de la protéine ERP mutée correspondante.

La présente invention a donc également pour objet une méthode de dépistage des individus auxquels a été administrée une composition vaccinale conforme à l'invention comprenant la mise en évidence de l'absence, dans un échantillon biologique desdits individus, d'anticorps ou de cellules T dirigés contre tout ou partie de la protéine ERP purifiée, l'échantillon biologique pouvant être du sang.

L'invention a également pour objet une composition comprenant la protéine ERP modifiée.

Un autre aspect de la présente invention concerne les séquences répétées présentent dans le gène *erp* notamment des souches du complexe *M. tuberculosis.* En effet, dans la majorité des cas étudiés par les Inventeurs, les patients tuberculeux ne développaient pas de réponse humorale contre *erp.* Les souris vaccinées par le BCG ne développent pas non plus de réponse humorale contre *erp.* A l'opposé, les patients lépromateux développent une importante réponse contre *erp.* La différence majeure entre la protéine ERP de *M. tuberculosis* et la protéine similaire *de M. leprae* réside dans l'absence de répétitions chez *M*. *leprae.*

Par conséquent, les répétitions pourraient être à l'origine du blocage de la réponse humorale spécifiquement contre *erp* ou même d'une façon générale contre d'autres antigènes. Il est en effet connu que les patients tuberculeux développent peu de réponse humorale au début de la tuberculose maladie. Il pourrait donc être possible d'utiliser les répétitions portées par *erp* pour inhiber le développement d'une réponse humorale spécifique en associant ces répétitions à tout antigène contre lequel on veut éviter qu'une réponse humorale ne soit induite ou peut être même utilisée ces répétitions pour inhiber toute réponse humorale dans certains contextes avantageux. Ce type de stratégie pourrait convenir aux situations suivantes : éviter le développement de la réponse humorale contre des vecteurs vaccinaux viraux. (voir tableau)

Ainsi, la présente invention concerne l'utilisation des séquences répétées du gène *erp,* facultativement en association avec au moins un autre antigène, pour inhiber le développement d'une réponse humorale.

Elle concerne également un vecteur d'expression dans un microorganisme, caractérisé par le fait qu'il comprend un séquence nucléotidique codant pour la protéine ERP dépourvue de ses séquences répétées. Le microorganisme hébergeant le vecteur d'expression peut par exemple être *E*. *coli* ou tout autre organisme susceptible de convenir à l'expression d'une séquence nucléotidique codant pour la protéine ERP dépourvue de ses séquences répétées, y compris les mycobactéries.

La présente invention a également pour objet une souche de mycobactéries caractérisée en ce que le gène *erp* est dépourvu de ses séquences répétées. En effet, une telle souche, présentant ERP sans répétition serait immunogène tout en ayant un effet protecteur.

De plus, la présente invention a pour objet une protéine recombinant ERP purifiée produite préférentiellement par *E*. *coli.* Avantageusement, cette protéine recombinante comprend six résidus histidine à son extrémité C-terminale.

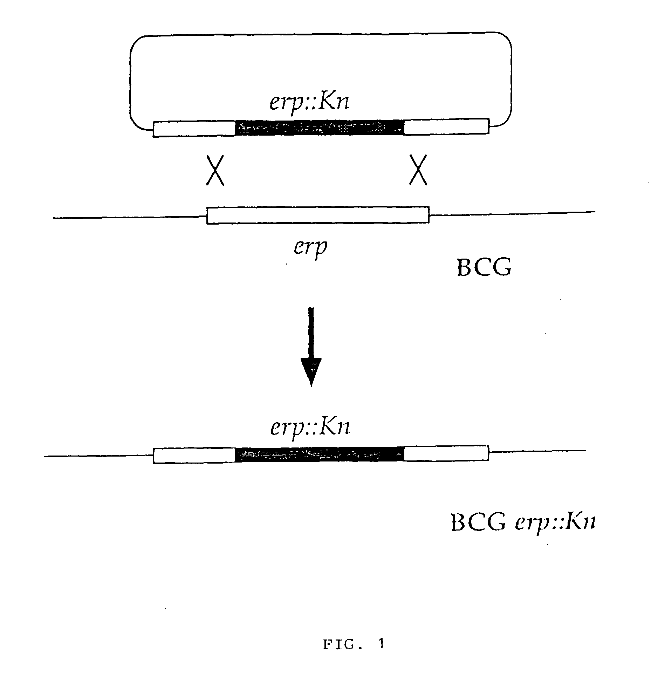

La figure 1 représente l'obtention d'une souche de BCG dont le gène *erp* a été inactivé par insertion d'une cassette de résistance à la kanamycine.
La figure 2 représente le nombre de cfu persistant dans chacun des organes concernés en fonction du nombre de jour suivant l'injection intraveineuse soit du BCG sauvage soit du BCG mutant (BCG *erp::Kn* aussi appelé BCG *erp::aph).*
La figure 3 représente le nombre de cfu résultant de la multiplication du BCG parental (1173P2) et du BCG muté *(erp::aph)* dans des cultures de macrophages dérivés de précurseurs médullaires de souris BALB/C.
La figure 4 représente le nombre de cfu résultant de la multiplication des souches parentales (wt), mutées (ERP') et complémentées de BCG et H37Rv dans des cultures de macrophages dérivés de précurseurs médullaires de souris Balb/c.
La figure 5 représente la comparaison du nombre de cfu résultant de la multiplication du BCG parental, muté et complémenté d'une part avec celle du H37Rv parental, muté et complémenté d'autre part, dans des cultures de macrophages dérivés de précurseurs médullaires de souris Balb/c, en fonction des organes (poumons, rate ou foie).

L'invention ne se limite pas à la description ci-dessus et sera mieux comprise à la lumière des exemples.

### Matérials et Méthodes

### Production et purification de protéine ERP recombinante

La région codant pour ERP privée de sa séquence signale a été amplifiée par PCR au moyen des amorces oligonucléotidiques His-2 (5'-AAGGAGATCTTGTGCATATTTTCTTGTCTAC-3') et His-3 (5'-AAGGAGATCTGGCGACCGGCACGGTGATTGG-3'), digérée par *Bg*/II et clônée dans le site *Bam*HI du plasmide d'expression pQE70 (QIAGEN GmbH, Hilden Allemagne). Le plasmide résultant, désigné pHis233, a subi une électroporation dans la souche M15 d'*Escherichia coli.*

Deux litres de cultures de la souche M15 d'*Esherichia coli* (pHis233) ont été mis à croître dans un bouillon Luria-Bertani, induits avec de l'ITPG et ont été traités au fin de purification protéique dans des conditions dénaturantes utilisant une résine agarose d'acide nickel-nitrilotriacétique (NTA) tel que décrit par le fournisseur (QIAGEN GmbH). ERP-His6 élué a été dialysé deux fois pendant 12 heures avec du PBS et stocké au froid à - 20°C. Deux lapins (souche de Nouvelle Zélande) ont été immunisés avec 100 µg de protéine et ensuite tous les quinze jours avec 150, 200 et 250 µg de ERP-His6 émulsifié dans l'adjuvant de Freund incomplet. Les sera anti-ERP hyper-immun ont été obtenus par la saignée des animaux six semaines après l'immunisation. La séparation par électrophorèse sous SDS-PAGE et l'immunoabsorption ont été réalisées comme décrit dans (J. Sambrook et al., 1987).

### Immunocytochimie (montage total)

Des cellules ont été fixées avec un tampon à 0,1 M de paraformaldéhydéine à 1 %, lavées dans le même tampon et ensuite appliquées à une grille de nickel revêtue de carbone Formvar, préalablement rendue hydrophile par le procédé électrique de « glow discharge ». Les grilles ont ensuite été préparées pour immunocytochimie, rincées avec de l'eau distillée et colorées négativement avec du molybdate d'amonium 1 % dans de l'eau.

### Cryosections

Les bactéries ou macrophages infectés (m.o.i. = 1) ont été fixés avec 2 % de paraformaldéhyde et 0,2 % de glutaraldéhyde dans 0,1 M de tampon phosphate. Les cellules ont été récoltées et emprisonnées dans de la gélatine à 10 %. Les cellules agglomérées ont été incubées de deux heures à toute une nuit dans du sucrose 1,8 et du pyrolidone de polyvinyl à 15 % (MW 10000). De petits blocs ont été montés sur des «porte-objets», refroidis dans l'azote liquide et cryosectionnés à -120°C avec un cryo-ultramicrotome Reickert FCS. Des sections minces ont ensuite été récupérées dans une goutte de sucrose 2,3 M et appliquées sur des grilles de nickel revêtues de carbone Formvar. Les grilles ont ensuite été traitées pour immunocytochimie, ensuite rincées avec de l'eau distillée et englobées dans de la méthylcellulose contenant de l'acétate d'uranyle à 0,3 %.

### Immunocytochimie

Des grilles ont été traitées avec des gouttes de réactifs suivants: NH₄Cl (50 mM) dans du PBS, 10 minutes, de l'Albumine de Sérum Bovin (BSA) 1 % (w/v) dans du PBS, 5 minutes, un anti-sérum anti-ERP dilué à 1/100 dans du PBS-BSA, 1 heure, PBS-BSA (trois lavages de 2 à 5 minutes chacun), conjugués à l'or anticorps IgG (chaînes H+L) anti-lapins (grains de 10 nm ou 5 nm en taille, British Biocell International, UK) dilués à 1/20 dans de la gélatine issue de peau de poisson PBS-0,1 % (Sigma), 30 à 45 minutes, PBS (un lavage, 1 minute) et eau distillée (trois lavages d'une minute chacun). Les exemples ont ensuite été fixés avec 1 % de glutaraldéhyde dans un tampon cacodylate 0,1 M (pH 7,4) pendant 2 minutes.

### Inactivation du gène erp

Un fragment d'ADN de 3,9 kb comprenant la longueur totale du gène *erp* a été coupé à partir de p1X412 par une digestion *Pst*I et cloné dans le site correspondant de pACYC 177. Le plasmide résultant désigné pB1a été linéarisé avec *Eco*R1 qui coupe à un site unique à l'intérieur de *erp.* En parallèle, une cassette *aph* conférant la résistance à la kanamycine a été coupée par digestion *Pst*I dans le plasmide pUC-4K. pPB1 et le fragment *aph* ont été coupé avec la polymérase à ADN T4 (Boehringer Mannheim) tel que recommandé par le fabricant, et ligaturé ensemble pour donner pPB2. Un fragment d'ADN de 5,2 kb contenant *erp: :aph* a été coupé de pPB2 par digestion *Pst*I et cloné dans pJQ200 non réplicatif donnant lieu au vecteur pPB3. Cinq µg de pPB3 ont subi une électroporation dans *M. bovi*s BCG qui a ensuite été étalé sur des plaques 7H11 Middelbrook supplémentées avec de la kanamycine (20 µg/ml). Des colonies ont été triées par PCR avec des oligonucléotides flanquant les sites *Eco*RI utilisés pour l'insertion d'*aph* et reportés (replica spotting) sur des plaques contenant de la kanamycine (20 g/ml) et 2 % de sucrose. Un clone contenant une insertion de 1,3 kb et plus sensible au sucrose a été analysé par Southern blot tel que décrit dans Berthet et al. 1995.

Des souris ont été maintenues et manipulées selon les directives de l'Institut Pasteur pour l'élevage d'animaux de laboratoires. Les macrophages issus de la moelle épinière ont été isolés à partir de fémures âgés de sept semaines, de souris femelles BALB/c. Les cellules ont été ensemencées à 5.10⁵ cellules par puits dans des chambres de cultures à 8 puits Labtek™ (Nunc) et ont été cultivées pendant sept jours tel que décrit dans Chastellier et al. 1995).

Le comptage des CFU a été réalisé tel que décrit dans Lagranderie et al. 1996.

### EXEMPLE 1 : Génération de sera polyclonaux de lapins anti-ERP

Quatre lapins Néozélandais femelles ont été immunisés une première fois avec 100µg de protéine ERP(his)6. Des immunisations secondaires (rappels) ont été effectués tous les 15 jours avec 150, 200 puis 250 µg de protéine purifiée. A partir de deux mois après la première immunisation, les sera collectés avaient un titre suffisant pour être utilisés pour l'immunodétection en Western blot et par immunohistochimie en microscopie électronique. Pour les expériences d'immunohistochimie en microscopie électronique, les séra ont été purifiés par adsorptiort/élution sur des bandelettes de nitrocellulose contenant ERP.

### EXEMPLE 2 : Construction d'une souche de BCG erp::Kn

Un fragment d'ADN de M. *tuberculosis* de 3,9kbp contenant le gène *erp* a été obtenu par digestion PstI du plasmide pIPX412 (CNCM No 1-1463) (Berthet et al., 1995). Ce fragment a été introduit dans le vecteur de clonage pACYC177, donnant naissance au plasmide pPB1. Le plasmide pPB1 a été ensuite linéarisé par l'enzyme de restriction *Eco*RI*,* coupant une seule fois au niveau d'un site situé dans le gène *erp.* En parallèle, une cassette *(aph)* génétique de petite taille (1,3kbp), conferrant la résistance à l'antibiotique kanamycine, a été préparée par restriction du plasmide pUC-4K par *Pst* I. Les fragments pACYC177/EcoRI et *aph*/*Pst I* ont été traités dans les conditions décrites par le fournisseur, par le fragment de Klenow de l'ADN polymerase 1 de *Escherichia coli* et par la polymerase du phage T4 respectivement, afin de générer des fragments aux extrémités franches. Ces deux fragments ont ensuite été ligaturés et transformés chez *E. coli.* Un plasmide recombinant ayant inséré la cassette *aph* au niveau du gène *erp* a été sélectionnée par hybridation sur colonies et dénommé pPB2. Le fragment contenant *erp::aph* a ensuite été excisé de pPB2 par digestion *PstI* et introduit dans le vecteur pJQ200 permettant éventuellement la contre sélection en présence de sucrose. Le plasmide résultant a été appelé pPB3. Le plasmide pPB3 (trois microgrammes) a été introduit dans la souche *Mycobacterium bovis* BCG Pasteur 1173 P2 par électroporation (Gene pulser BioRad, 2500V, 200Ω, 25 µF). Les cellules transformées ont été incubées durant 24 heures à 37°C dans du milieu 7119 (5ml) puis étalées sur des boites 7H11 contenant de la kanamycine (2µg/ml). Les boites ont été incubées durant 25 jours à 37°C et trente colonies de bactéries résistantes à la kanamycine ont été repiquées individuellement à la fois par PCR en utilisant un couple d'oligonucléotides flanquant le site *Eco*RI du gène *erp,* et par Southern blot en utilisant une sonde interne de *erp.* Un clone recombinant, dénommé BCG *erp::Kn,* a été sélectionné sur les critères suivants:
1- Par PCR, disparition de la bande correspondant à l'allèle sauvage (500bp) pour le BCG *erp::Kn* mutant. Obtention d'un fragment d'amplification PCR de 1800bp (500+1300) signant l'insertion de la cassette *aph* dans la copie génomique du gène *erp*
2- Par Southern blot, détection d'un signal d'hybridation à 5.2kbp avec l'ADN du BCG 13K au lieu de 3.9kbp pour le BCG sauvage. Perte du site *EcoRI* interne au gène *erp::aph.*

### EXEMPLE 3 : Test de la persistance du BCG erp : :aph chez la souris.

Un stock bactérien conservé à -70°C de la souche BCG *erp::Kn a* été réalisé de la manière suivante: une colonie poussée sur 7H11+Kn (20µg/ml) a été ensemencée sur milieu pomme de terre/sauton en présence de kanamycine (20µg/ml) jusqu'à formation d'un voile. Ce voile a servi à réaliser un inoculum (10µg/ml) pour des ballons contenant du milieu sauton liquide. Après 8 jours de croissance, le voile ainsi formé en sauton est récupéré, broyé et est resuspendu dans du milieu Beck-Proskauer additionné de glycérol 6% (Vol./Vol.). Le stock ainsi obtenu titrait 4,8 10⁸ unités formant des colonies (CFU) /ml.

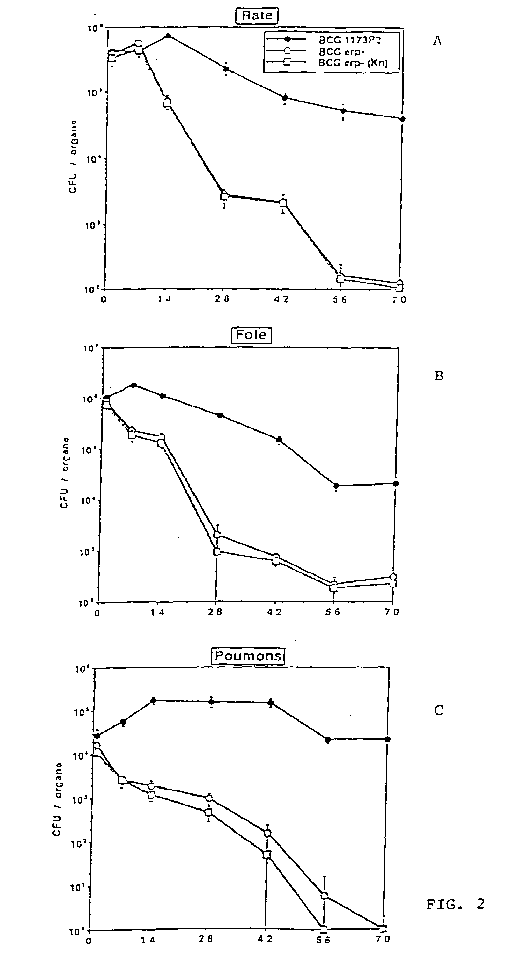

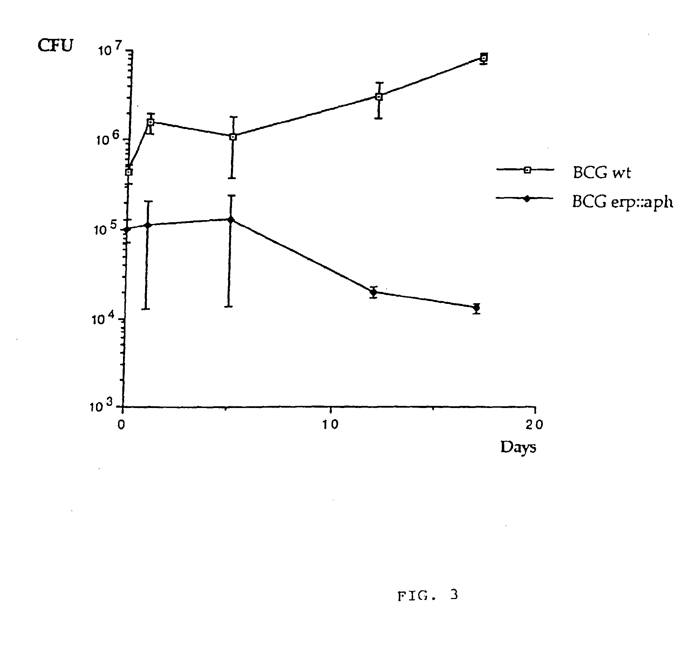

A l'aide de ce stock, des souris BALB/c ont été injectées par voie intraveineuse avec 10⁶ cfu de BCG sauvage et mutant (BCG *erp::Kn)* en suspension dans du PBS. Trois organes, la rate, le foie et les poumons, ont été prélevés stérilements aux jours 1, 7, 14, 28, 42, 56 et 70. A chaque point, les organes ont été broyés en milieu Beck-Proskauer et les bactéries ont été énsemencées à différentes dilutions sur des boites 7H11 avec ou sans kanamycine (20µg/ml). Le nombre de bactéries viables présentent dans les différents organes en fonction du temps a été déterminé par le comptage des CFU (Figure 2).

### EXEMPLE 4 : Etude de la multiplication du BCG erp::Kn dans des macrophages dérivés de précurseurs médullaire de souris.

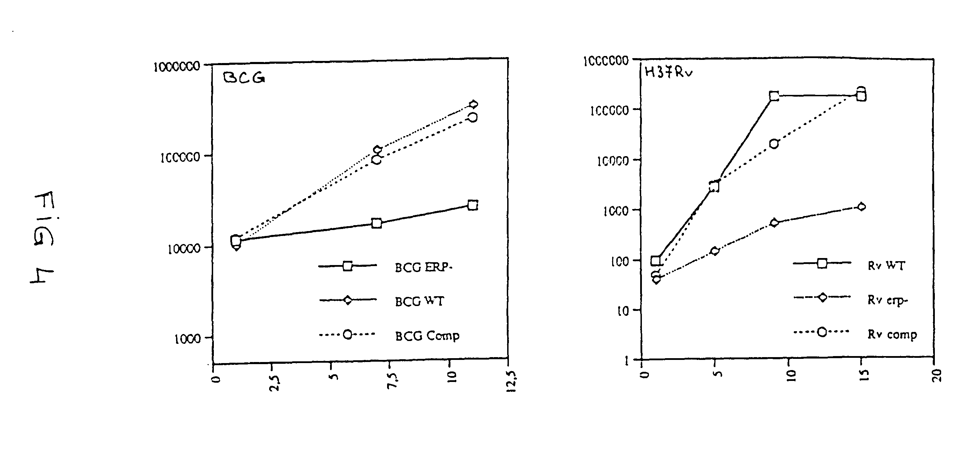

Des expériences précédentes ont montré que la souche BCG *erp::Kn* ne persiste plus chez la souris. L'organe dans lequel l'élimination du BCG erp::Kn est la plus marquée est le poumon. Les macrophages alvéolaires représentent la cible primaire de l'infection par les mycobactéries du complexe *M. tuberculosis.* Afin de préciser le type cellulaire dans lequel la persistance du BCG *erp::Kn* est affectée, nous avons étudié la multiplication de cette souche dans des macrophages dérivés de précurseurs médullaire (BMDP) de souris. Pour celà, des BMDP issus de fémur de souris femelles BALB/CBYJICO agées de 7 semaines ont été isolés et mis en culture (milieu DMEM (Gibco (BRL), Glutamine 2 mM, Sérum de veau foetal (Dominique Deutscher SA) 10% Vol./Vol., Surnageant de cellules L229 10%) dans des chambres Labtek™ 8 puits, à une densité cellulaire de 5.10⁴ cellules dans 400µl de milieu. Les cellules ont été cultivées durant 7 jours avant d'être infectées durant quatre heures par du BCG 1173 P2 (souche parentale) ou du BCG *erp::Kn* (souche mutée) à une multiplicité d'infection de 1. A différents temps post-infection (Jour 0, Jour 1, Jour 5, Jour 12, Jour 17) les macrophages infectées ont été lysés dans un tampon préservant l'intégrité des mycobactéries et le lysat obtenu a été étalé sur des boites de milieu 7H11 à différentes dilution (de 10⁰ à 10⁻⁶). Les boites de pétri ont été incubées à 37°C durant un mois afin de mesurer l'évolution du nombre d'unités formant des colonies représenté figure 3.

### EXEMPLE 5 : Etude de la réaction d'hypersensibilité retardée induite chez le cobaye par le BCG erp::kn.

La réaction d'hypersensibilité retardée (DTH) reflète l'induction de réponses immunitaires dirigées contre des composantes mycobactériens. Une induration d'un diamètre supérieur à une valeur seuil, provoquée par l'injection intradermique de tuberculine signe un contact au préalable avec des mycobactéries. Ce test permet un diagnostic rapide de l'infection tuberculeuse chez les sujets non vaccinés. Cependant ce test est difficilement utilisable chez les sujets vaccinés par le BCG qui sont alors positifs. Nous avons testé la capacité du BCG *erp::Kn* à induire une réaction DTH. Pour celà, deux groupes de 5 cobayes (mâles de 300gr, souche Dunkin Hartley) ont été immunisés par 5.10⁵ unité viables de BCG 1173P2 ou *erp::Kn* respectivement. Un mois plus tard, les mêmes animaux ont été immunisés par voie intradermique avec les préparation suivantes:
* "Purified Protein derivative" (PPD)/ tuberculine (WEYBRIDGE) 2µg
* Protéine 65kDa *M. leprae* purifiée 50ug et 100µg

Le diamètre de l'induration a été mesurée 48h après l'injection des différents antigènes sur chacun des 5 animaux constituant le groupe. Une induration positive supérieure à 8-10 mm est considérée comme positive.

Il a été observé que le BCG *erp::Kn* induisait une DTH après immunisation par la PPD et la protéine 65kD de *M. leprae.*

### EXEMPLE 6 : Caractérisation génotypique et phénotypique du BCG erp::Kn.

La caractérisation du mutant *M*. *bovis* BCG *erp::Kn* a été entreprise de deux manières. Dans un premier temps, l'ADN génomique du BCG mutant (M) a été extrait et analysé par la technique hybridation moléculaire de Southern (Berthet et al., 1995) en comparaison avec l'ADN génomique de la souche de BCG parentale (P). Une digestion par EcoRI indique que le génome du BCG *erp::Kn* a perdu un tel site, localisé dans le gène *erp* et détruit par l'insertion de la cassette kanamycine. De plus une digestion par PstI, indique que le fragment de restriction portant *erp* chez BCG *erp::Kn* comporte une insertion de 1,3 kbp correspondant à la présence de la cassette kanamycine. Ces données confirment le remplacement de l'allèle sauvage *erp* par un allèle muté *erp::Kn* chez BCG *erp::Kn.*

Dans un second temps, l'expression de la protéine ERP a été analysée chez le BCG sauvage et chez le BCG *erp::Kn* par immunodétection selon la méthode dite de «Western blot» (Sambrook et al., 1989) à l'aide d'un sérum de lapin anti-ERP. La protéine ERP n'est plus détectable dans le surnageant du BCG *erp::Kn.*

### EXEMPLE 7: Préparation de la souche H37RV de Mycobacterium tuberculosis : H37RV erp: :kn

La souche H37RV *erp : :kn* dérive de la souche de référence *Micobacterium tuberculosis.* La souche a pour particularité de ne plus produire la protéine correspondant au gène *erp.* Le gène *erp* détermine la synthèse d'une protéine exportée répétitive localisée à la surface des bactéries complexes *Micobacterium tuberculosis.* Cette souche a été construite au cours d'une expérience de recombinaison homologue par remplacement de la copie sauvage du gène *erp* par une copie mutée, en utilisant le plasmide pIPX56 comme décrit précédemment. La version mutée du gène *erp* contient une insertion d'une cassette conférant la résistance à la kanamycine au niveau du site de restriction *Eco*RI*.* Une telle mutation abolit la synthèse du gène d'une protéine *erp* fonctionnelle. Un des phénotypes associé à l'introduction de cette mutation est la perte de la capacité de persister chez la souris.

### EXEMPLE 8: Caractérisation du produit du gène erp de Mycobacterium tuberculosis

Pour caractériser le produit du gène *erp* de *M. tuberculosis*, la protéine recombinante ERP a été purifiée et surproduite. La protéine a été synthétisée dans *E. coli* en fusion avec 6 résidus histidine (ERP-6His). ERP-6His forme des corps d'inclusion cytoplasmic et est ensuite purifiée par immobilisation sur chromatographie d'affinité au nickel dans des conditions dénaturantes. Renaturée, ERP-6His soluble est analysée par un gel d'électrophorèse à deux dimensions. (Laurent-Winter, 1997)

ERP-6His est séparée en deux espèces ayant le même poids moléculaire (36 kDa) mais différent en ce qui concerne leur point isoélectrique (pI). La forme majoritaire a un pI de 5,3 qui correspond à celui calculé pour ERP-6His. La forme minoritaire est plus acide (pI 5,2) est susceptible de correspondre à une forme aberrante apparaissant dans le cytoplasme de *E*. *coli.* Cette préparation de ERP-6His a été utilisée pour immuniser des lapins et un sérum polyclonal fortement titré a été obtenu. Au moyen de ce sérum, des bandes immunoréactives de 36 et 34 kDa ont été détectées à la fois dans les fractions associées aux cellules et au filtrats de culture précipités par le TCA (acide trichloracétique) de BCG et de *M*. *tuberculosis* (souche Mt 103). La bande supérieure a co-migré avec ERP-6His recombinante. La bande 34 kDa pourrait être le résultat d'une dégradation protéolytique ou alternativement d'un traitement post-traductionnel ayant lieu chez *M*. *tuberculosis.* Ces données sont en accord avec celles montrant que l'antigène PGLTS, une protéine de *M.bovis* ayant plus de 99% d'identité avec la protéine ERP de *M. tuberculosis,* est présente sous forme d'un doublet de MW similaire dans des fluides cellulaires concentrés (BIGI et al 1995).

Sur la base des caractéristiques structurelles, il a déjà été avancé que la protéine ERP peut également être présente à la surface des bactéries. Pour déterminer précisément la localisation subcellulaire de ERP, le bacille de *M. tuberculosis* fixé a été mis en contact avec un sérum anti-ERP et ensuite incubé avec un conjugué anti-lapin marqué à l'or. L'observation par transmission en microscopie électronique a révélé un marquage de surface intense à la périphérie du bacille, indiquant que ERP est une molécule exposée à la surface. Ce résultat a été confirmé par l'observation de la paroi cellulaire marquée sur des tuyaux section de *M.tuberculosis* (données non montrées).

Il a ensuite été déterminé si ERP était produite durant la multiplication intra cellulaire de *M. tuberculosis* à l'intérieur des macrophages cultivés. Dans ce but, des macrophages de souris J774 ont été infectés avec un isolat clinique de *M. tuberculosis* et ont ensuite été observés par microscopie immunoélectronique.

Tandis qu'aucun marquage significatif n'a été observé avec le sérum pré-immun de ERP, un marquage spécifique de la paroi cellulaire mycobactérienne est du lumen phagosomal a été observé avec le sérum de lapin immunisé par ERP. De plus, de petites vésicules contenant ERP marqué ont été observées dans l'environnement proche des phagosomes. Ceci démontre que ERP est produite dans les phagosomes de *M. tuberculosis* et suggère que ERP se déplace à l'intérieur des cellules.

### EXEMPLE 9: Rôle de la protéine ERP dans la croissance intra cellulaire des mycobactéries

Il a ensuite été examiné si ERP était un composant bactérien essentiel pour l'étape de croissance intra cellulaire. Dans ce but, une mutation nulle ciblée a été introduite dans le locus *erp* de la souche H37Rv de *M. tuberculosis* et dans la souche BCG de *M. bovis* et dans la souche vaccinale modèle de *M. bovis* BCG. Un vecteur suicide à contre sélection par le sucrose pJQ200 a été utilisé pour introduire un allèle mutant de *erp (erp::aph)* dans le chromosome de *M. bovis* BCG.

*M. tuberculosis* correspondante a été construite utilisant la technologie *ts-sacB* (Pelisic et al., 1997).

Des souches mutantes résultant de l'échange allélique ont été appelées BCG *erp::aph* et H37Rv *erp::aph.* Au moyen du vecteur intégratif dérivé du mycobactériophage MS6 (pAV6950) une simple copie de *erp* a été réintroduite au site attB de BCG *erp::aph* et H37Rv *erp::aph.* L'analyse de l'ADN chromosomique extrait de la souche parental (P), mutante (M) ou complémentée (par complémenté, on entend la réintroduction d'un gène *erp* fonctionnel capable de diriger la synthèse de la protéine ERP) a révélé que le site *Eco*R1 coupé durant la construction de *erp::aph* était également perdu dans le génome des souches mutantes et complémentées. De plus, l'analyse utilisant *PstI* indique une insertion de 1,3 kb à l'intérieur du fragment de restriction portant *erp*. L'hybridation de la même membrane avec les séquences des vecteurs pJQ200 et pPR27 n'a pas permis de détecter un quelconque signal (données non montrées) suggérant que seule la cassette *erp::aph* était introduite dans le génome de BCG *erp::aph* et H37Rv *erp::aph.* L'analyse des fractions associées aux cellules et des surnageants concentrés à partir des cultures de BCG *erp::aph* et H37Rv *erp::aph* a indiqué que l'interruption de *erp* avait aboli la production de ERP. Ceci a été confirmé par microscopie immunoelectronique par la disparition du marquage à l'or sur les cryosections BCG *erp::aph* de *M. bovis.* Au contraire, l'intégration de *erp* au site attB des souches mutantes *erp::aph* a restauré la production de ERP, à la fois à la surface des cellules et dans le milieu de culture de *M. tuberculosis* et *M., bovis.* L'analyse morphologique de la colonie, doublant la durée du temps et les caractéristiques de croissance dans la culture 7H9/ADC du Middelbrook ou du milieu Sauton minimum, n'a pas permis d'identifier une quelconque différence entre les souches mutantes parentales et complémentées de BCG et de H37Rv. Prises ensemble, ces données montrent que *erp* n'est pas essentiel à la croissance de BCG et H37Rv dans des conditions de laboratoire.

### EXEMPLE 10 :

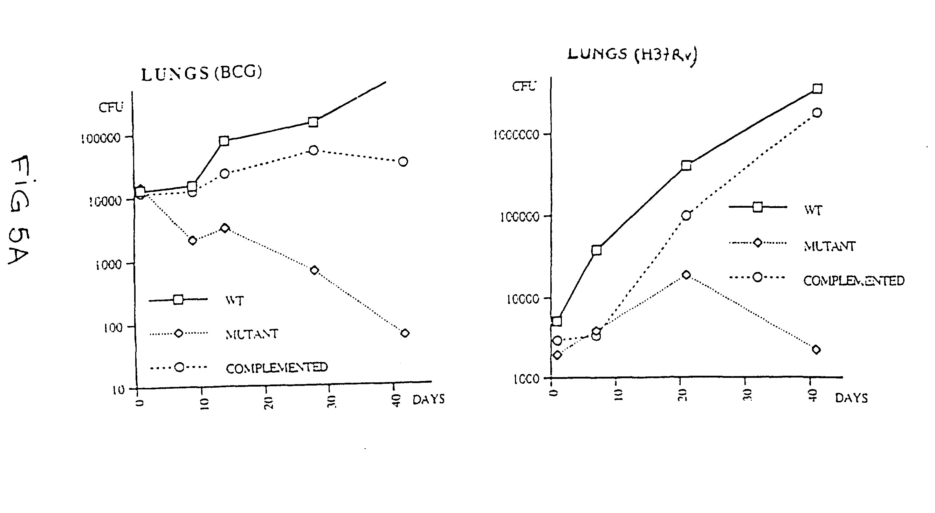

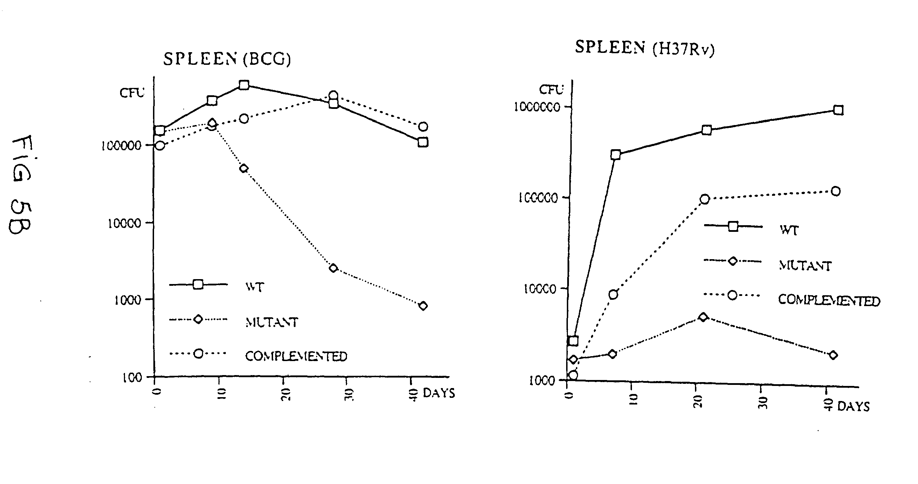

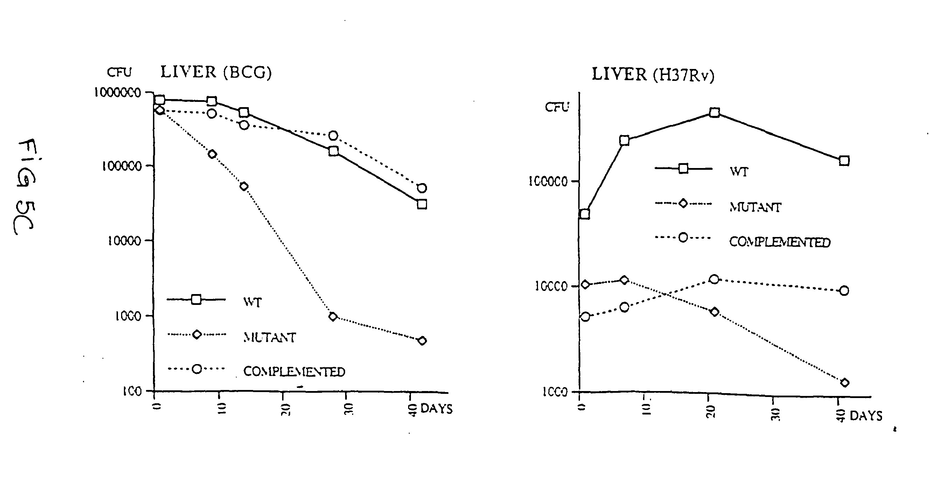

La capacité de BCG *erp : :aph* et H37Rv *erp : :aph* à croître au sein des cellules phagocytiques a été examinée. Dans ce but, la multiplication des souches mutantes et parentales dans une culture de macrophages dérivés de moelle cellulaire a été comparée. Comme montré à la figure 4A, le dénombrement des CFU indique les mutants *erp : :aph* ne se multiplient pas au sein des macrophages de souris tandis que les souches parentales et complémentées ont une croissance normale. De plus, la souche H37Rv *erp : :aph* montre une réduction des effets cytopathiques comparés aux souches parentales et complémentées (figure 4B). Pour déterminer si la mutation *erp : :aph* affecte également la multiplication au sein de l'hôte, la persistance de BCG *erp : :aph* et H37Rv *erp ::aph* dans les souris a été analysée. 106 unités viables des souches parentales, mutantes *erp : :aph* et erp-complémentées ont été injectées par voie intra-veineuse à des souris Balb/c et l'infection bactérienne a été contrôlée par le comptage des CFU au delà d'une période de 56 jours (Lagranderie et al., 1996). Le comptage a été réalisé dans les poumons, le foie et la rate, trois organes connus pour contenir la plus importante charge microbactérienne après inoculation par voie intra-veineuse. Tel que représenté dans la figure 5A, les mutants BCG *erp: :aph* ont été rapidement éliminés des poumons des animaux infectés tandis que les souches correspondantes parentales et complémentées ont colonisé ce tissu et ont survécu. Au contraire, le mutant H37Rv *erp : :aph* a survécu mais s'est multiplié très lentement en comparaison avec les souches parentales et complémentées. Les poumons représentent le site d'infection par les membres du complexe *M. tuberculosis* durant la tuberculose. La multiplication des mutants *erp : aph* a également fortement diminuée dans le foie (figure 5B) et la rate (figure 5C). De plus, la morphologie des colonies BCG après avoir infecté un animal est très différente : tandis que le BCG parental donne lieu à une morphologie de colonies dites « diffuses », le BCG *erp : :aph* ne diffuse plus et montre une croissance retardée (jusqu'à une semaine comparée à la souche parentale). La signification de cette observation est inconnue mais la perte du phénotype « diffus » a été corrélée avec les niveaux les plus bas de virulences résiduelles parmi les sous-souches BCG (Dubos et Pierce, 1956, Pierce et Dubos, 1956, Pierce Dubos et Scheiffer, 1956 et Dubos et Pierce, 1956). Le phénotype « non diffus » n'est pas permanent et est perdu après restriction du milieu de culture sur 7H11. De plus, la réintroduction de *erp* restaure le phénotype parental. Quoiqu'il en soit, ces données démontrent que l'expression *erp* est requise durant l'étape de croissance intra-cellulaire des mycobactéries appartenant au complexe *M. tuberculosis.*

**TABLEAU**

| Origine | Nb de sera testés | MBP-ERP* | ERP-His6 | 65kDa BCG |
|---|---|---|---|---|
| Humains Contrôlés | 4 | - | - | +/- |
| Humains (Bligny) Tuberculeux *M. tuberculosis* | 21 | - | - | + (En Pool) |
| Humains (Ouganda) Tuberculeux *M. tuberculosis* | 10 | ND | +++(3/10) | ND |
| Enfants (Necker) Tuberculeux *M. tuberculosis* (Exam. Direct +) | 4 | - | - | + (En Pool) |
| Humains Madagascar Tuberculeux *M. bovis* | 6 | - | - | + |
| Humains (Népal) Lépreux lépromateux *M. leprae* | 1 Pool | +++ | +++ | +++ |
| Bovins Tuberculeux *M. bovis* | 4 | +++ | +++ | +++ |

| | | | | |
|---|---|---|---|---|
| * Protéine ERP en fusion avec la Maltose Binding Protein | | | | |

Le sérum des individus testés (ou le pool dans le cas de lépreux) a été mis en contact avec chacune des trois protéines mentionnées dans le tableau. La réponse immunitaire a été mesurée.

### REFERENCES

Arruda, S., Bornfim, G., Knights, R., Huima-Byron, T. & Riley, L. W. (1993) Science 261: 1454-1457.
Azad, A. K., Sirakova, T.D., Rogers, L. M. & Kolattukudy, P.B. (1996) Proc. Nat. Acad. Sci. USA 93:4787-4792.
Balasubramanian, V., Pavelka Jr., M.S., Bardarov, S.S., Martin, J., Weisbrod, T.R., McAdam, R.A., Bloom, B.R. & Jacobs Jr, W.R. (1996) J. Bacteriol. 178:273-279.
Balasuc A.-M., Deriaud E., Leclerc C. D., Georghiu M. Infect. Immunn. 64, 1 (1996).
Berthet, F.X., Rauzier J., Lim, E.M., Philipp, W., Gicquel B. and D. Portnoi (1995) Characterization of the Mycobacterium tuberculosis erp gene encoding a potential cell surface protein with repetitive strutures. Microbiology 141:2123-2310.
Bigi F., Alito A., Fisanotti J. C., Romano M. I., Cataldi A., Infect. Immun. 63, 2581 (1995).
Bloom, B.R. & Murray, C.J.L. (1992) Science 257:1055-1064.
Chastellier de C., Lang T., Thilo L., Eur. J. Cell Biol. 68, 167 (1995).
Collins, D.M., Kawakami, R.P., de Lisle, G.W., Pascopella, L., Bloom, B.R. & Jacobs Jr, W. R. (1995) Proc. Nat. Acad. Sci. USA 92:8036-8040.
Curtiss, III, Roy, (1990) New Generation Vaccines, pp. 161-165.
Dooley, S. W., Jarvis, W.R., Martone, W.J. and D.E. Snider (1992). Multidrug resistant tuberculosis. Ann. Intern. Med. 117:257-259.
Dubos R. J., Pierce C. H., Amer. Rev. Tuberc. 74, 655-666, (1956).
Dubos R.J., Pierce C. H., ibid. 74, 699-717, (1956).
Falkow, S. (1988) Rev. Infect. Dis. 10:5274-5276.
Guilhot, C., Otal, I., van Rompaey, I., Martin, C. & Gicquel, B. (1994) J. Bacteriol. 176:535-539.
Hoiseth, Susan K. & Stocker, B.A. D., (1981) Nature, 291:238-239.
Jacobs Jr., W.R. (1992) Immunobiol. 184:147-156.
Lagranderie M.R., Balazuc A.-M., Deriaud E., Leclerc C.D., Georghiu M., Infect. Immun. 64, 1 (1996).
Laurent-Winter C., Ngo S., Danchin A., Bertin P., Eur. J. Biochem. 244, 767 (1997).
Levin, M.M., Herrington, D., Murphy, J.R., Morris, J.G., Losonsky, G., Tall, B., Lindberg, A., Norman, B., Dellagostin, O.A., McFadden, J. & Dale, J.W. (1995) Mol. Microbiol. 16:755-760.
Noriega, Fernando R., Wang, J.Y., Losonsky, G., Maneval D.R., Hone, D.M. & Levin, M.M., (1994) Infect. Immum., 62 (11):5168-5172.
Oyston, P.C.F., Willimson, E.D., Leary, S.E.C., Eley, S.M., Griffin, K.F. & Titball, R.W., (1995) Infec. Immun., 63(2):563-568.
Pascopella, L., Collins, F.M., Martin, J.M., Lee, M.H., Hatftill, G.F., Stover, C.K., Bloom, B.R. & Jacobs Jr., W.R. (1994) Infect.Immun. 62:1313-1319.
Pelicic, V., Reyrat, J.-M. & Gicquel, B. (1996) FEMS Microbiol. Lett. 144:161-166.
Pelicic V., Jackson M., Reyrat J.M., Jacobs W.R., Gickel B. and Guilhot C., 1997, Efficient allelic exchange and tranposon mutagenesis in M. tuberculosis, PROC. NATL. ACAD. SCIE. USA, vol 94, 10955-10960.
Pierce C. H., Dubos R. J., ibid. 74, 667-682, (1956).
Pierce C. H., Dubos R. J.,Schaefer W. B., ibid. 74, 683-698, (1956).
Reyrat, J.-M., Berthet, F.-X. & Gicquel, B. (1995) Proc. Nat. Acad. Sci. USA 92:8768-8772.
Sambrook, J. Fritsch, E.F., and T. Maniatis. 1989. Molecular cloning: a laboratory manual. 2ed Cold Spring Harbor Laboralory, Cold spring Harbor, New York*.*
Sambrook J., Frietsch E.F., Maniatis T., Molecular cloning: a laboratory manual (Cold Spring Harbor Laboratory Press, 1987) pp. 18.47-18.74 [Second édition].
Straley, Susan C. and Harmon, Paula A, (1984) Infection and Immunity, 45(3):649-654.
Sudre, P., Ten Dam, G. and A. Kochi (1992). La tuberculose aujourd'hui dans le monde. Bull. WHO 70 (3):297-308.

## Revendications

1. Souche de *Mycobacterium* dont le gène *erp* a subi une modification abolissant la production de la proteine correspondante ou permettant la production d'une proteine différent d'au moins 20% en terme d'activité par rapport à la proteine naturelle, par insertion d'un polynucléotide comprenant un gène de sélection.

2. Souche de *Mycobacterium* selon la revendication 1, **caractérisée en ce que** le gène de sélection code pour la résistance à un antibiotique.

3. Souche de *Mycobacterium* selon la revendication 2, **caractérisée en ce que** l'antibiotique est la kanamycine, la spectinomycine ou l'hygromycine.

4. Souche de *Mycobacterium* selon l'une des revendications 1 à 3, **caractérisée en ce qu'**elle appartient au genre *Mycobacterium,* de préférence au complexe *M. tuberculosis.*

5. Souche de *Mycobacterium* selon la revendication 4, **caractérisée en ce qu'**elle appartient à l'espèce *Mycobacterium tuberculosis* ou à l'espèce *Mycobacterium bovis.*

6. Souche de *Mycobacterium* selon la revendication 5, **caractérisée en ce qu'**elle correspond à la souche BCG *erp::Kn* (CNCM No I-1896) ou un variant incapable d'exprimer le produit du gène *erp* actif.

7. Souche de *Mycobacterium* selon la revendication 5, **caractérisée en ce qu'**elle comprend la souche H37Rv *erp: :aph* (CNCM N° 1-2048) ou un variant incapable d'exprimer le produit du gène *erp* actif.

8. Souche de *Mycobacterium* selon l'une des revendications 1 à 5, **caractérisée en ce qu'**elle est capable d'exprimer un polynucléotide codant pour un antigène de mycobactérie d'une autre espèce que celle à laquelle ladite souche appartient.

9. Souche de *Mycobacterium* selon l'une des revendications 1 à 5, **caractérisée en ce qu'**elle est capable d'exprimer un polynucléotide étranger au genre *Mycobacterium.*

10. Vecteur recombinant comprenant tout ou partie du gène *erp* modifié par insertion d'un polynucléotide comprenant un gène de sélection.

11. Vecteur recombinant selon la revendication 10, **caractérisé en ce qu'**il comprend une origine de replication thermosensible chez les mycobactéries.

12. Vecteur recombinant selon la revendication 10 ou 11, **caractérisé en ce qu'**il comprend le gène *sacB.*

13. Vecteur recombinant selon la revendication 10, **caractérisé en ce que** le gène de sélection code pour la résistance à un antibiotique.

14. Vecteur recombinant selon la revendication 13, **caractérisé en ce que** l'antibiotique est la kanamycine, la spectinomycine ou l'hygromycine.

15. Vecteur recombinant selon l'une des revendications 10 à 14, **caractérisé en ce qu'**il comprend un insert correspondant au gène *erp* modifié et qu'il s'agit de pIPX56 (CNCM No I-1895).

16. Utilisation d'un vecteur recombinant selon l'une des revendications 10 à 15 pour la préparation par échange allélique d'une souche de *Mycobacterium* selon l'une des revendications 1 à 9.

17. Procédé pour la production d'une souche de *Mycobacterium* selon l'une des revendications 1 à 9, comprenant les étapes de :
- transformation avec un vecteur recombinant selon l'une quelconque des revendications 10 à 15, d'une souche de *Mycobacterium* propagée à une température permissive,
- mise en culture des colonies résultant de la transformation sur un milieu supplémenté avec un produit de sélection et du saccharose,
- isolement de la souche ainsi sélectionnée.

18. Procédé selon la revendication 17, **caractérisé en ce que** le produit de sélection est un antibiotique choisi parmi la kanamycine, la spectinomycine et l'hygromycine

19. Composition immunogène comprenant une souche de *Mycobacterium* selon l'une des revendications 1 à 9.

20. Composition vaccinale comprenant une souche de *Mycobacterium* selon l'une des revendications 1 à 9, en combinaison avec au moins un excipient pharmaceutiquement compatible.

## Claims

1. *Mycobacterium* strain whose *erp* gene has undergone a modification abolishing the production of the corresponding protein or allowing the production of a protein which is at least 20% different in terms of activity compared with the natural protein, by insertion of a polynucleotide comprising a selectable gene.

2. *Mycobacterium* strain according to Claim 1, **characterized in that** the selectable gene encodes the resistance to an antibiotic.

3. *Mycobacterium* strain according to Claim 2, **characterized in that** the antibiotic is kanamycin, spectinomycin or hygromycin.

4. *Mycobacterium* strain according to one of Claims 1 to 3, **characterized in that** it belongs to the *Mycobacterium* genus, preferably to the *M. tuberculosis* complex.

5. *Mycobacterium* strain according to Claim 4, **characterized in that** it belongs to the *Mycobacterium tuberculosis* species or to the *Mycobacterium bovis* species.

6. *Mycobacterium* strain according to Claim 5, **characterized in that** it corresponds to the BCG *erp::Kn* strain (CNCM No. 1-1896) or a variant incapable of expressing the product of the active *erp* gene.

7. *Mycobacterium* strain according to Claim 5, **characterized in that** it comprises the H37Rv *erp::aph* strain (CNCM No. 1-2048) or a variant incapable of expressing the product of the active *erp* gene.

8. *Mycobacterium* strain according to one of Claims 1 to 5, **characterized in that** it is capable of expressing a polynucleotide encoding an antigen of the mycobacterium of a species other than that to which said strain belongs.

9. *Mycobacterium* strain according to one of Claims 1 to 5, **characterized in that** it is capable of expressing a polynucleotide foreign to the *Mycobacterium* genus.

10. Recombinant vector comprising the whole or part of the *erp* gene is modified by insertion of a polynucleotide comprising a selectable gene.

11. Recombinant vector according to Claim 10, **characterized in that** it comprises a replication origin which is heat-sensitive in mycobacteria.

12. Recombinant vector according to Claim 10 or 11, **characterized in that** it comprises the *sacB* gene.

13. Recombinant vector according to Claim 10, **characterized in that** the selectable gene encodes the resistance to an antibiotic.

14. Recombinant vector according to Claim 13, **characterized in that** the antibiotic is kanamycin, spectinomycin or hygromycin.

15. Recombinant vector according to one of Claims 10 to 14, **characterized in that** it comprises an insert corresponding to the modified *erp* gene and **in that** it is pIPX56 (CNCM No. 1-1895).

16. Use of a recombinant vector according to one of Claims 10 to 15 for the preparation, by allelic exchange, of a *Mycobacterium* strain according to one of Claims 1 to 9.

17. Method for the production of a *Mycobacterium* strain according to one of Claims 1 to 9 comprising the steps of:
- transforming, with a recombinant vector according to any one of Claims 10 to 15, a *Mycobacterium* strain propagated at a permissive temperature,
- culturing the colonies resulting from the transformation on a medium supplemented with a selectable product and sucrose,
- isolating the strain thus selected.

18. Method according to Claim 17, **characterized in that** the selectable product is an antibiotic chosen from kanamycin, spectinomycin and hygromycin.

19. Immunogenic composition comprising a *Mycobacterium* strain according to one of Claims 1 to 9.

20. Vaccine composition comprising a *Mycobacterium* strain according to one of Claims 1 to 9, in combination with at least one pharmaceutically compatible excipient.

## Patentansprüche

1. *Mycobacterium-*Stamm, bei welchem das *erp*-Gen eine Modifizierung durchlaufen hat, welche die Produktion des entsprechenden Proteins vernichtet oder die Produktion eines Proteins, welches sich zu wenigstens 20% in Hinblick auf die Aktivität bezogen auf das natürliche Protein unterscheidet, erlaubt, durch Insertion eines Polynukleotids, welches ein Selektionsgen umfasst.

2. *Mycobacterium-*Stamm nach Anspruch 1, **dadurch gekennzeichnet, dass** das Selektionsgen die Resistenz gegen ein Antibiotikum kodiert.

3. *Mycobacterium-Stamm* nach Anspruch 2, **dadurch gekennzeichnet, dass** das Antibiotikum Kanamycin. Spectinomycin oder Hygromycin ist.

4. *Mycobacterium-*Stamm nach einem der Ansprüche 1 bis 3, **dadurch gekennzeichnet, dass** er zu der Gattung *Mycobacterium,* vorzugsweise zu dem *M*. *tubaroulosis*-Komplex gehört.

5. *Mycobacterium-*Stamm nach Anspruch 4, **dadurch gekennzeichnet, dass** er zu der Spezies *Mycobacterium tuberculosis* oder zu der Spezies *Mycobacterium bovis* gehört.

6. *Mycobactefiurn-Stamm* nach Anspruch 5, **dadurch gekennzeichnet, dass** er dem Stamm BCG *erp::Kn* (CNCM Nr. 1-1896) oder einer Variante, welche nicht in der Lage ist, das aktive Produkt des *erp*-Gens zu exprimieren, entspricht.

7. *Mycobacterium-Stamm* nach Anspruch 5, **dadurch gekennzeichnet, dass** er den Stamm H37Rv *erp::aph* (CNCM Nr. 1-2048) oder eine Variante, welche nicht in der Lage ist, das aktive Produkt des *erp*-Gens zu exprimieren, umfasst.

8. *Mycobacterium-*Stamm nach einem der Ansprüche 1 bis 5, **dadurch gekennzeichnet, dass** er in der Lage ist, ein Polynukleotid zu exprimieren, welches ein Antigen von Mycobakterien einer anderen Spezies als jener, zu der der Stamm gehört, kodiert.

9. *Mycobacterium-*Stamm nach einem der Ansprüche 1 bis 5, **dadurch gekennzeichnet, dass** er in der Lage ist, ein Polynukleotid zu exprimieren, das bezogen auf die Gattung *Mycobacterium* fremd ist.

10. Rekombinanter Vektor, welcher die Gesamtheit oder einen Teil des *erp*-Gens, welches durch Insertion eines Polynukleotids, welches ein Selektionsgen umfasst, modifiziert worden ist, umfasst.

11. Rekombinanter Vektor nach Anspruch 10, **dadurch gekennzeichnet, dass** er einen in den Mycobakterien wärmeempfindlichen Replikationsstartpunkt umfasst.

12. Rekombinanter Vektor nach Anspruch 10 oder 11, **dadurch gekennzeichnet, dass** er das Gen *sacB* umfasst.

13. Rekombinanter Vektor nach Anspruch 10, **dadurch gekennzeichnet, dass** das Selektionsgen die Resistenz gegen ein Antibiotikum kodiert.

14. Rekombinanter Vektor nach Anspruch 13, **dadurch gekennzeichnet, dass** das Antibiotikum Kanamycin, Spectinomycin oder Hygromycin ist.

15. Rekombinanter Vektor nach einem der Ansprüche 10 bis 14, **dadurch gekennzeichnet, dass** er ein Insert, welches dem modifizierten erp-Gen entspricht, umfasst und dass es sich um pIPX56 (CNCM Nr. 1-1895) handelt.

16. Verwendung eines rekombinanten Vektors nach einem der Ansprüche 10 bis 15 für die Herstellung eines *Mycobacterium*-Stammes gemäß einem der Ansprüche 1 bis 9 durch Allelaustausch.

17. Verfahren zur Herstellung eines *Mycobacterium-*Stammes nach einem der Ansprüche 1 bis 9, welches die Schritte umfasst:
- Transformation eines bei einer permissiven Temperatur vermehrten *Mycobacterium-*Stammes mit einem rekombinanten Vektor nach einem der Ansprüche 10 bis 15,
- In-Kultur-Nehmen der aus der Transformation resultierenden Kolonien auf einem Medium, welches mit einem Selektionsprodukt und Saccharose ergänzt ist,
- Isolierung des so selektierten Stamms.

18. Verfahren nach Anspruch 17, **dadurch gekennzeichnet, dass** das Selektionsprodukt ein unter Kanamycin, Spectinomycin und Hygromycin ausgewähltes Antibiotikum ist.

19. Immunogene Zusammensetzung, umfassend einen *Mycobacterium-*Stamm nach einem der Ansprüche 1 bis 9.

20. Impfzusammensetzung, umfassend einen *Mycobacterium-*Stamm nach einem der Ansprüche 1 bis 9 in Kombination mit wenigstens einem pharmazeutisch verträglichen Träger.
